# EUROPEAN PATENT SPECIFICATION

(11) **EP 2 586 393 B1**
(45) Date of publication and mention of the grant of the patent: **08.10.2014**
(21) Application number: 11187116.6
(22) Date of filing: 28.10.2011
(51) Int. Cl.: A61B 17/72, A61B 17/86

(54) **Locking hole arrangement**
Verriegelungslochanordnung
Verrouillage d'agencement de jet

(43) Date of publication of application: 01.05.2013
(73) Proprietor: Stryker Trauma GmbH, 24232 Schönkirchen/Kiel (DE)
(72) Inventor: Wieland, Manfred, 24146 Kiel (DE); Petersen, Stephan, 24105 Kiel (DE); Zander, Nils, 24340 Eckernförde (DE)
(74) Representative: Schmitz, Alexander

(56) References cited:
- WO-A2-2007/124099
- WO-A2-2011/002903
- US-B1- 6 221 074

## Description

### FIELD OF THE INVENTION

The invention generally relates to an intramedullary nail. Particularly, the invention relates to an intramedullary nail and a locking member, wherein the intramedullary nail comprises a locking hole arrangement for angularly stable receipt of the locking member.

### BACKGROUND OF THE INVENTION

In general, an intramedullary nail may be for example a femur nail, a humerus nail or a tibia nail. Usually, the leading end portion which may at first be introduced into a medullary channel of a bone and which may also be denoted as non-driving end portion of the intramedullary nail, comprises two or more through holes adapted to receive locking screws.

At present inserting locking screws in holes formed in the leading end of implanted intramedullary nails is problematic, namely because of the amount of radiation required during the determination of the position and orientation of transverse locking holes. Furthermore, it is time consuming and ideally requires well-trained and experienced personal. Therefore, it has a significant influence of the overall operation room time required.

Currently, locking of the non-driving end portion of an intramedullary nail is performed mostly freehand, by inserting a first locking screw into and through a first through hole. Due to the fact that the insertion is performed more or less blindly, the screw will in most cases not exactly hit the through hole so that the nail will be slightly displaced or even deformed to accommodate the locking screw extending through the through hole. However, this first screw will be able to move relative to the nail when forces are applied on the nail and screw.

For a locking providing angular stability, a second screw may be inserted through a second through hole adjacent the first hole. Also the second screw will in most cases not exactly hit the second through hole. Accordingly, the second screw has to be urged through the hole, with the result that the combination of the first and second screws will provide an angle-stable fixation of the intramedullary nail, i.e. a fixation with no movement of a screw relative to the nail.

However, such a fixation of an intramedullary nail requires the insertion of two screws, wherein each insertion of a screw through the leading end portion of an intramedullary nail is difficult and thus time consuming.

According to EP 1 440 664 B1, a deformable ring may be arranged between a threaded bore and the thread of a bone screw to achieve an angle-stable locking. WO 2005/079685 A1 proposes a deformable sleeve on a locking screw, for introducing the screw backlash-free into a transverse hole of a marrow nail. Alternatively, US 2006/0064095 A1 proposes a passage extending through a screw head and an external thread generally parallel the longitudinal axis of the screw for accommodating a longitudinal wedge element. As a result, any gap between the locking screw and an intramedullary nail is eliminated and the screw is wedged in position in a transverse hole of the intramedullary nail.

WO 2007/124099 A2 discloses an intramedullary nail according to the preamble of claim 1.

### SUMMARY OF THE INVENTION

It may be seen as a need to make locking of the non-driving end portion of intramedullary nails easier. In general, it is of interest to shorten the operation room time which is beneficial not only for the patient under anaesthesia, but ultimately reduces costs.

This is achieved by the subject-matter of the independent claim. Further embodiments are described in the respective dependent claims.

In general, an intramedullary nail according to the invention comprises a longitudinal nail axis, a first lateral portion, a second lateral portion opposite to the first lateral portion, a longitudinal bore, and a locking hole arrangement having a first bore with a first bore axis, wherein the first bore is formed in the first lateral portion and communicates with the longitudinal bore, and a second bore with a second bore axis, wherein the second bore is formed in the second lateral portion and communicates with the longitudinal bore. The first bore axis and the second bore axis are arranged with an offset between each other, and are arranged so that a locking member is insertable through both the first bore and the second bore.

An intramedullary nail with a longitudinal bore, i.e. with a through bore extending along a longitudinal axis of the intramedullary nail, may also be denoted as cannulated intramedullary nail, or as a hollow nail.

A lateral portion of a hollow or cannulated intramedullary nail may be understood as a portion forming substantially half of the circumference of the nail and extending in a longitudinal direction of the nail. In other words, a first and a second lateral portion may be achieved by cutting an intramedullary nail along its longitudinal axis.

According to an embodiment of the invention, the first bore comprises a first bore diameter and the second bore comprises a second bore diameter, wherein the offset is smaller than 20 percent of the first bore diameter and is smaller than 20 percent of the second bore diameter, wherein the first bore diameter may be equal to the second bore diameter.

According to another embodiment of the invention, the first bore axis is arranged parallel to the second bore axis. It will be understood that the two axes may also be orientated inclined relative to each other, wherein the axes may or may not intersect each other.

According to an embodiment of the invention, the first bore axis and the second bore axis are arranged with an offset in a direction of the nail axis. According to a further embodiment of the invention, the first bore axis and the second bore axis are arranged with an offset perpendicular to the nail axis. It will be understood that the intended angle-stable fixation may also be achieved with two bores the bore axes of which are arranged with an offset in any direction other than the direction of the nail axis or the direction perpendicular to the nail axis, i.e. inclined to these directions. In other words, the offset may have a component both in the direction of the nail axis and in the direction perpendicular to the nail axis.

According to a further embodiment of the invention, the first bore axis and the second bore axis intersect the nail axis.

According to another embodiment of the invention, the first bore axis and the second bore axis are arranged perpendicular to a plane including the nail axis. This means that a bore axis may pass the nail axis without intersecting the same.

For example, the first bore axis may pass the nail axis with a distance of 0,3mm on a first side of the axis, and the second bore axis may pass the nail axis with a distance of 0,3mm on a second side, i.e. opposite side, of the nail axis.

According to an embodiment of the invention, the first bore diameter is smaller than 60 percent of a nail diameter and the second bore diameter is also smaller than 60 percent of the nail diameter.

A longitudinal bore diameter may be smaller than 60 percent of a nail diameter. Furthermore, a longitudinal bore axis may be coaxial or congruent with the longitudinal nail axis.

For example, the nail diameter may be 10mm, the diameter of the first and second bores may be 5,1mm, and the diameter of the longitudinal bore may be 5mm. For accommodating a 5mm universal screw, an appropriate offset may be 0,6mm in this example.

According to a further embodiment of the invention, a system includes an intramedullary nail and a first locking member having a first locking member diameter, wherein the first locking member diameter is smaller than the first bore diameter and greater than the first bore diameter minus the offset, and is smaller than the second bore diameter and greater than the second bore diameter minus the offset.

It is noted that, although a screw may be a preferred locking member, also a bolt or nail may be use as locking member, since also a locking member without threads may frictionally engage within the first and second bores. A nail or bolt may have, instead of threads, a smooth outer surface or alternatively a rough surface, wherein a rough surface may be a surface formed with any kind of notches or impressions.

In case the locking member is a screw, it will be understood that a first screw thread of the first locking member may be formed so that the first screw thread can frictionally engage within the first and second bores, due to the offset between these bores.

The term 'frictionally engage' should be understood as firmly fixed in a transverse direction, i.e. not enabling any movement of the locking member transverse to its axis relative to the nail, but allowing the locking member to be moved in a longitudinal direction of the locking member, for example by screwing the locking member in or out of the hole arrangement.

When for example a screw is screwed in a hole arrangement, the screw thread may cause a deformation of an edge of at least one of the bores, or the screw thread may cut into a surface of at least one of the bores, so that a well defined connection between the screw and the nail is formed. In this case, the material of the screw is harder than the material of the nail.

According to yet another embodiment of the invention, the material of the nail is harder than the material of the locking member so that the locking member or at least a part of the outer surface of the locking member will be deformed when the locking member is introduced through the locking hole arrangement, wherein said outer surface may be defined by threads of a screw.

According to yet a further embodiment of the invention, a system with the intramedullary nail may further comprise a second locking member having a second locking member diameter, wherein the second locking member diameter is smaller than the first bore diameter minus the offset and smaller than the second bore diameter minus the offset.

It should be pointed out that the locking member or screw denoted as first locking member or first screw is adapted for an angle-stable fixation of the nail, and that the locking member or screw denoted as second locking member or second screw is adapted for a non-angle-stable fixation of the nail.

Furthermore, it is noted that a system according to the invention may comprise an intramedullary nail including at least one locking hole arrangement, together with at least one first locking member or first screw and/or at least one second locking member or second screw, for example depending on the preferences of a physician utilizing the system.

The aspects defined above and further aspects, features and advantages of the present invention can also be derived from the examples of the embodiments to be described hereinafter and are explained with reference to examples of the embodiments to which the invention is not limited.

### BRIEF DESCRIPTION OF THE DRAWINGS

The invention will now be detailed by way of an exemplary embodiment with reference to the attached drawings.
Fig. 1 illustrates a section view of an intramedullary nail together with a first screw for angle-stable locking of the nail and a second screw for non-angle-stable locking of the nail, according to the invention.
Fig. 2 is a schematical illustration of a locking hole arrangement of an intramedullary nail according to the invention.

It is noted that the illustration in the drawings is only schematically and not to scale. In different figures, similar elements are provided with the same reference signs.

### DETAILLED DESCRIPTION OF AN EXEMPLARY EMBODIMENT

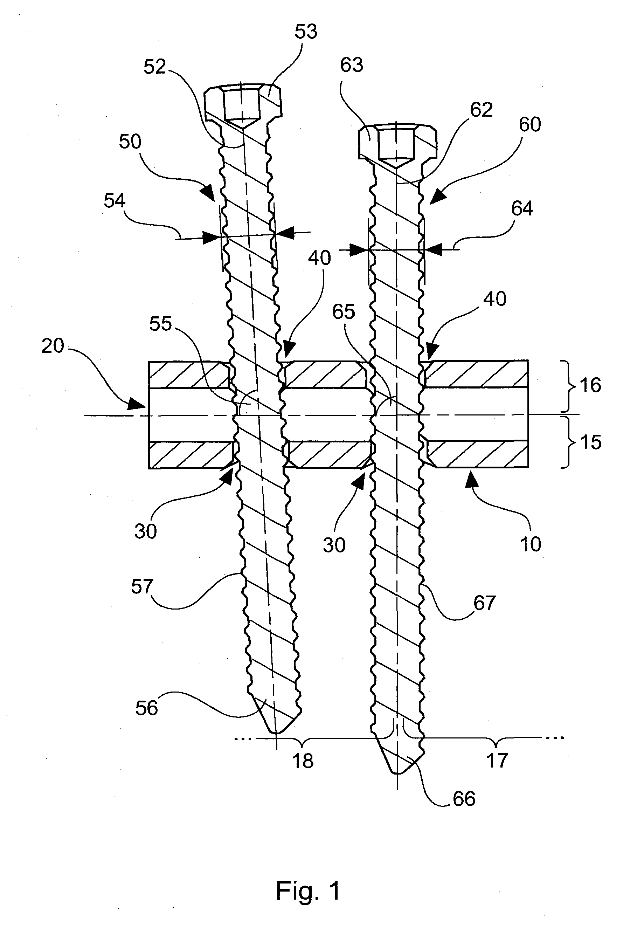

Fig. 1 is an illustration showing a section of an intramedullary nail 10 with a longitudinal bore 20. The nail comprises a first lateral portion 15, i.e. substantially the lower half of the nail, and a second lateral portion 16, i.e. substantially the upper half of the nail (as depicted in fig. 1). Further, the nail 10 comprises two hole arrangements each including a first bore 30 formed in the first lateral portion 15, and a second bore 40 formed in the second lateral portion 16.

In fig. 1, a first screw 50 is inserted into the left hole arrangement. The first screw 50 includes a first screw head 53, a first screw tip 56 and a first screw thread 57. Further, the first screw 50 comprises a first screw axis 52 and a first screw diameter 54. It will be understood that the screw diameter is an overall outer diameter of the screw.

The first screw 50 is adapted for an angle-stable fixation of the nail 10. Accordingly, the first screw diameter 54, the form and dimension of the first screw thread 57, as well as the diameters of the first and second bores 30, 40, and the offset between these bores are closely interrelated or interdependent to each other. Therefore, the first screw 50 is arranged with an angle 55 relative to the longitudinal axis of the nail 10, wherein the angle 55 may be orthogonal to the nail axis, but will in most cases not be orthogonal to the nail axis.

Also in fig. 1, a second screw 60 is shown, inserted into the right hole arrangement. The second screw 60 includes a second screw head 63, a second screw tip 66 and a second screw thread 67. Further, the second screw 60 comprises a second screw axis 62 and a second screw diameter 64.

The second screw 60 is adapted for a non-angle-stable fixation of the nail 10. Accordingly, the second screw diameter 64, as well as the diameters of the first and second bore 30, 40, together with the offset between these bores are also but not in the same way as the first screw closely interrelated and interdependent to each other. Therefore, the second screw 60 is arranged with an angle 65 relative to the longitudinal axis of the nail 10, which angle 65 may be, in many cases, orthogonal to the nail axis.

To achieve on the one hand an angle-stable fixation and on the other hand a non-angle-stable fixation, the first screw diameter 54 may be greater than the second screw diameter 64. As can be seen in fig. 1, the first bore 30 and the second bore 40 of the left hole arrangement are substantially occupied by the first screw 50, whereas the first bore 30 and the second bore 40 of the right hole arrangement are not completely filled by the second screw 60, but leave a small area free, i.e. on the right side in the first bore 30 and on the left side in the second bore 40.

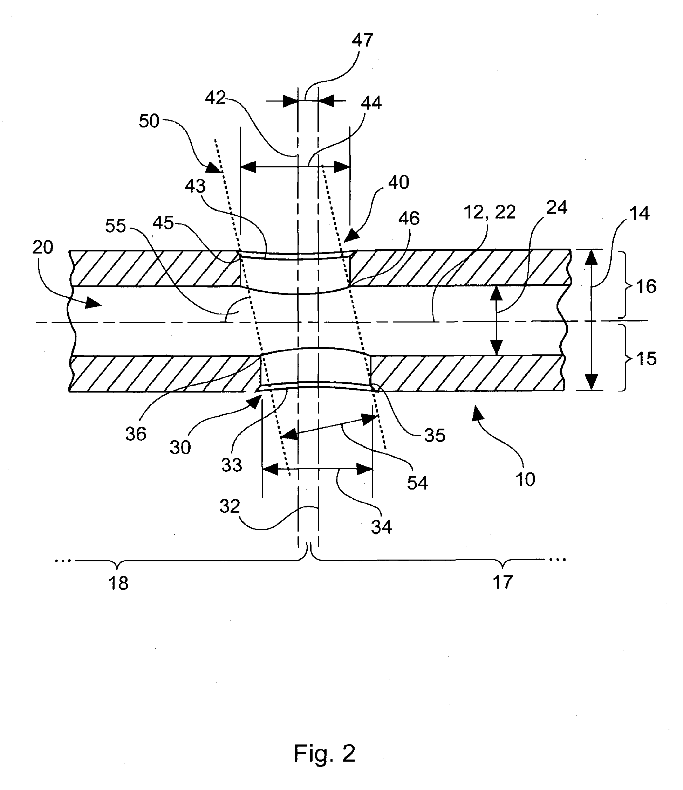

Fig. 2 is an illustration of the cannulated intramedullary nail 10 having a longitudinal bore 20, a first bore 30 in a first lateral portion 15 and a second bore 40 in a second lateral portion 16.

The nail 10 includes a longitudinal nail axis 12 and a nail diameter 14. The longitudinal bore 20 is defined by the longitudinal bore axis 22 and the longitudinal bore diameter 24. In this exemplary embodiment, the longitudinal nail axis 12 is coaxial or congruent to the longitudinal bore axis 22.

The first bore 30 has a first bore axis 32 and a first bore diameter 34. Correspondingly, the second bore 40 comprises a second bore axis 42 and a second bore diameter 44. Furthermore, a first bore chamfer 33 is formed as a transition from the surface of the first bore 30 to the outer surface of the nail 10, i.e. to the outer surface of the first lateral portion 15 of the nail 10, and a second bore chamfer 43 is formed as a transition from the surface or the second bore 40 to the outer surface of the nail 10, i.e. to the outer surface of the second lateral portion 16 of the nail.

Further illustrated in fig. 2 is an offset 47 between the first bore axis 32 and the second bore axis 42. In this exemplary embodiment, the offset 47 is arranged substantially in a direction of the longitudinal axis 12 of the nail 10.

In dashed lines, a first locking member 50 which may be a bolt, nail or a screw, is illustrated extending through both the first bore 30 and the second bore 40. The first locking member diameter 54 is smaller than the first and second bore diameters 32, 42, but due to the offset 47, the outer edges of the first locking member engage a first contact point 35, a second contact point 36, a third contact point 45 and a fourth contact point 46. It will be understood that each contact point includes an area defined by a section of an edge together with a section of the wall surface adjacent this edge of a respective bore.

In the exemplary embodiment of fig. 2, the first contact point 35 is at an outer edge of the first bore, at the first lateral portion 15 and at a first longitudinal portion 17 of the nail 10. The second contact point 36 is at an inner edge of the first bore 30, at the first lateral portion 15 and at a second longitudinal portion 18 of the nail 10. The third contact point 45 is at the outer edge of the second bore 40, at the second lateral portion 16 and at the second longitudinal portion 18 of the nail. The fourth contact point 46 is at an inner edge of the second bore 40, at the second lateral portion 16 and at the first longitudinal portion 17 of the nail. Thus, the first screw 50 is positioned with an inclined angle 55 relative to the longitudinal nail axis 12.

The first locking member / screw, the left one in fig. 1, may deform the outer edge of the first bore at the first contact point, may deform the inner edge of the first bore at the second contact point, may cut into the side wall of the second bore at the third contact point, and may deform and cut the inner edge of the second bore at the fourth contact point.

In a first step, an intramedullary nail according to the invention may be implanted into a bone canal. It is noted, that an intramedullary nail is usually implanted to fix at least two fractured bone fragments relative to each other. Accordingly, locking screws are used at both ends of the intramedullary nail, each fixing a bone fragment relative to the nail.

In a next step, a locking member, like a locking screw, is inserted in a transverse direction to the nail through a first bone portion and into a first bore formed in the nail. By further driving in of the locking member, the tip of the locking member will also be inserted into a second bore formed with an offset and opposite to the first bore. Due to the offset, a force will act between the locking member and the walls of the bores.

The force acting between the locking member and the walls of the bores subsequently deforms at least one of the locking member and the walls of the bores, depending on the respective hardness of these elements. For example, when the locking member is made from a material which is harder than the material of the nail, substantially the walls of the bores will be deformed. On the other hand, when the nail is made from a material which is harder than the material of the locking member, substantially the outer surfaces of the locking member, for example the threads of a locking screw, will be deformed, wherein also a bending of the shaft of the locking member may occur. It will be understood, that the locking member as well as the walls of the bores will be deformed, when the respective materials comprise a similar hardness.

By way of this deformation, an accurate fitting engagement between the locking member and the nail will be achieved, being a force fit engagement.

While the invention has been illustrated and described in detail in the drawings and foregoing description, such illustration and description are to be considered illustrative or exemplary and not restrictive. The invention is not limited to the disclosed embodiments.

Other variations to the disclosed embodiments can be understood and effected by those skilled in the art in practising the claimed invention, from the study of the drawings, the disclosure and the appended claims. In the claims, the word "comprising" does not exclude other elements and indefinite article "a" or "an" does not exclude a plurality. The mere fact that certain measures are recited in mutual different dependent claims does not indicate that a combination of these measures cannot be used to advantage. Any reference signs in the claims should not be construed as limiting the scope.

### List of reference signs

- 10: intramedullary nail
- 12: nail axis
- 14: nail diameter
- 15: first lateral portion
- 16: second lateral portion
- 17: first end portion
- 18: second end portion
- 20: longitudinal bore
- 22: longitudinal bore axis
- 24: longitudinal bore diameter
- 30: first bore
- 32: first bore axis
- 33: first bore chamfer
- 34: first bore diameter
- 35: first contact point
- 36: second contact point
- 40: second bore
- 42: second bore axis
- 43: second bore chamfer
- 44: second bore diameter
- 45: third contact point
- 46: fourth contact point
- 47: offset between first and second bore axes
- 50: first locking member / first screw
- 52: first locking member axis / first screw axis
- 53: first locking member head / first screw head
- 54: first locking member diameter / first screw diameter
- 55: angle between first locking member axis / first screw axis and nail axis
- 56: first locking member tip / first screw tip
- 57: first screw thread
- 60: second locking member / second screw
- 62: second locking member axis / second screw axis
- 63: second locking member head / second screw head
- 64: second locking member diameter / second screw diameter
- 65: angle between second locking member axis / second screw axis and nail axis
- 66: second locking member tip / second screw tip
- 67: second screw thread

## Claims

1. An intramedullary nail (10), the nail comprising
a longitudinal nail axis (12),
a first lateral portion (15),
a second lateral portion (16) opposite to the first lateral portion,
a longitudinal bore (20),
a first bore (30) with a first bore axis (32) wherein the first bore is formed in the first lateral portion and communicates with the longitudinal bore, and
a second bore (40) with a second bore axis (42), wherein the second bore is formed in the second lateral portion and communicates with the longitudinal bore,
wherein the first bore axis and the second bore axis are arranged so that a single locking member is insertable through both the first bore and the second bore,
**characterized in that** the first bore axis and the second bore axis are arranged with an offset (47) between each other.

2. The intramedullary nail (10) according to claim 1, wherein the first bore (30) comprises a first bore diameter (34) and the second bore (40) comprises a second bore diameter (44), wherein the offset (47) is smaller than 20 percent of the first bore diameter (34) and is smaller than 20 percent of the second bore diameter (44).

3. The intramedullary nail (10) according to claim 2, wherein the first bore diameter (34) is equal to the second bore diameter (44).

4. The intramedullary nail (10) according to any one of claims 1 to 3, wherein the first bore axis (32) is arranged parallel to the second bore axis (42).

5. The intramedullary nail (10) according to any one of claims 1 to 4, wherein the first bore axis (32) and the second bore axis (42) are arranged with an offset (47) in a direction of the nail axis (12).

6. The intramedullary nail (10) according to any one of claims 1 to 5, wherein the first bore axis (32) and the second bore axis (42) are arranged with an offset (47) in a direction perpendicular to the nail axis (12).

7. The intramedullary nail (10) according to any one of claims 1 to 6, wherein the first bore axis (32) and the second bore axis (42) are arranged perpendicular to a plane including the nail axis (12).

8. The intramedullary nail (10) according to any one of claims 1 to 7, wherein at least one of the first bore axis (32) and the second bore axis (42) intersect the nail axis (12).

9. The intramedullary nail (10) according to any one of claims 1 to 8, wherein the first bore diameter (34) is smaller than 60 percent of a nail diameter (14), and wherein the second bore diameter (44) is smaller than 60 percent of the nail diameter (14).

10. The intramedullary nail (10) according to any one of claims 1 to 9, wherein a longitudinal bore diameter (24) is smaller than 60 percent of a nail diameter (14).

11. The intramedullary nail (10) according to any one of claims 1 to 10, wherein a longitudinal bore axis (22) is congruent with the longitudinal nail axis (12).

12. A system comprising
an intramedullary nail (10) according to any one of claims 1 to 11, and
a first locking member (50) having a first locking member diameter (54),
wherein the first locking member diameter is smaller than the first bore diameter (34) and greater than the first bore diameter minus the offset (47), and is smaller than the second bore diameter (44) and greater than the second bore diameter minus the offset (47).

13. The system according to claim 12, wherein the first locking member (50) is a screw.

14. The system according to claim 13, wherein the screw (50) includes a first screw thread (57) which is formed so that the first screw thread can frictionally engage with both the first bore and the second bore.

15. The system according to any one of claims 12 to 14, wherein the first locking member (50) has a first locking member material and the nail (10) has a nail material, wherein the first locking member material is harder than the nail material.

16. The system according to any one of claims 12 to 14, wherein the first locking member (50) has a first locking member material and the nail (10) has a nail material, wherein the nail material is harder than the first locking member material.

17. The system according to any one of claims 12 to 16, further comprising a second locking member (60) having a second locking member diameter (64), wherein the second locking member diameter is smaller than the first bore diameter (34) minus the offset (47) and smaller than the second bore diameter (44) minus the offset (47).

18. A system comprising
an intramedullary nail (10) according to any one of claims 1 to 11, and
a second locking member (60) having a second locking member diameter (64), wherein the second locking member diameter is smaller than the first bore diameter (34) minus the offset (47) and smaller than the second bore diameter (44) minus the offset (47).

19. The system of claim 17 or 18, wherein the second locking member is a screw.

## Patentansprüche

1. Intramedullärer Nagel (10), wobei der Nagel Folgendes umfasst:
eine Nagellängsachse (12),
einen ersten Seitenabschnitt (15),
einen zweiten Seitenabschnitt (16) gegenüber dem ersten Seitenabschnitt,
eine Längsbohrung (20),
eine erste Bohrung (30) mit einer ersten Bohrungsachse (32), wobei die erste Bohrung im ersten Seitenabschnitt gebildet ist und mit der Längsbohrung in Verbindung steht, und
eine zweite Bohrung (40) mit einer zweiten Bohrungsachse (42), wobei die zweite Bohrung im zweiten Seitenabschnitt gebildet ist und mit der Längsbohrung in Verbindung steht,
wobei die erste Bohrungsachse und die zweite Bohrungsachse derart angeordnet sind, dass ein einzelnes Verriegelungselement durch sowohl die erste Bohrung als auch die zweite Bohrung verlaufend eingefügt werden kann,
**dadurch gekennzeichnet, dass** die erste Bohrungsachse und die zweite Bohrungsachse mit einem Versatz (47) zueinander angeordnet sind.

2. Intramedullärer Nagel (10) nach Anspruch 1, wobei die erste Bohrung (30) einen ersten Bohrungsdurchmesser (34) umfasst und die zweite Bohrung (40) einen zweiten Bohrungsdurchmesser (44) umfasst, wobei der Versatz (47) kleiner als 20 Prozent des ersten Bohrungsdurchmessers (34) und kleiner als 20 Prozent des zweiten Bohrungsdurchmessers (44) ist.

3. Intramedullärer Nagel (10) nach Anspruch 2, wobei der erste Bohrungsdurchmesser (34) gleich dem zweiten Bohrungsdurchmesser (44) ist.

4. Intramedullärer Nagel (10) nach einem der Ansprüche 1 bis 3, wobei die erste Bohrungsachse (32) parallel zur zweiten Bohrungsachse (42) angeordnet ist.

5. Intramedullärer Nagel (10) nach einem der Ansprüche 1 bis 4, wobei die erste Bohrungsachse (32) und die zweite Bohrungsachse (42) mit einem Versatz (47) in Richtung der Nagelachse (12) angeordnet sind.

6. Intramedullärer Nagel (10) nach einem der Ansprüche 1 bis 5, wobei die erste Bohrungsachse (32) und die zweite Bohrungsachse (42) mit einem Versatz (47) in einer Richtung senkrecht zur Nagelachse (12) angeordnet sind.

7. Intramedullärer Nagel (10) nach einem der Ansprüche 1 bis 6, wobei die erste Bohrungsachse (32) und die zweite Bohrungsachse (42) senkrecht zu einer Ebene angeordnet sind, welche die Nagelachse (12) einschließt.

8. Intramedullärer Nagel (10) nach einem der Ansprüche 1 bis 7, wobei die erste Bohrungsachse (32) und/oder die zweite Bohrungsachse (42) die Nagelachse (12) schneiden.

9. Intramedullärer Nagel (10) nach einem der Ansprüche 1 bis 8, wobei der erste Bohrungsdurchmesser (34) kleiner als 60 Prozent eines Nageldurchmessers (14) ist und wobei der zweite Bohrungsdurchmesser (44) kleiner als 60 Prozent des Nageldurchmessers (14) ist.

10. Intramedullärer Nagel (10) nach einem der Ansprüche 1 bis 9, wobei ein Längsbohrungsdurchmesser (24) kleiner als 60 Prozent eines Nageldurchmessers (14) ist.

11. Intramedullärer Nagel (10) nach einem der Ansprüche 1 bis 10, wobei eine Längsbohrungsachse (22) kongruent mit der Nagellängsachse (12) ist.

12. System, Folgendes umfassend:
einen intramedullären Nagel (10) nach einem der Ansprüche 1 bis 11 und
ein erstes Verriegelungselement (50), das einen ersten Verriegelungselement durchmesser (54) aufweist,
wobei der erste Verriegelungselementdurchmesser kleiner als der erste Bohrungsdurchmesser (34) und größer als der erste Bohrungsdurchmesser abzüglich des Versatzes (47) ist sowie kleiner als der zweite Bohrungsdurchmesser (44) und größer als der zweite Bohrungsdurchmesser abzüglich des Versatzes (47) ist.

13. System nach Anspruch 12, wobei das erste Verriegelungselement (50) eine Schraube ist.

14. System nach Anspruch 13, wobei die Schraube (50) ein erstes Schraubengewinde (57) beinhaltet, das derart gebildet ist, dass das erste Schraubengewinde reibschlüssig sowohl mit der ersten Bohrung als auch mit der zweiten Bohrung sein kann.

15. System nach einem der Ansprüche 12 bis 14, wobei das erste Verriegelungselement (50) ein erstes Verriegelungselementmaterial aufweist und der Nagel (10) ein Nagelmaterial aufweist, wobei das erste Verriegelungselementmaterial härter als das Nagelmaterial ist.

16. System nach einem der Ansprüche 12 bis 14, wobei das erste Verriegelungselement (50) ein erstes Verriegelungselementmaterial aufweist und der Nagel (10) ein Nagelmaterial aufweist, wobei das Nagelmaterial härter als das erste Verriegelungselementmaterial ist.

17. System nach einem der Ansprüche 12 bis 16, ferner ein zweites Verriegelungselement (60) umfassend, das einen zweiten Verriegelungselementdurchmesser (64) aufweist, wobei der zweite Verriegelungselementdurchmesser kleiner als der erste Bohrungsdurchmesser (34) abzüglich des Versatzes (47) und kleiner als der zweite Bohrungsdurchmesser (44) abzüglich des Versatzes (47) ist.

18. System, Folgendes umfassend:
einen intramedullären Nagel (10) nach einem der Ansprüche 1 bis 11 und
ein zweites Verriegelungselement (60), das einen Verriegelungselementdurchmesser (64) aufweist, wobei der zweite Verriegelungselementdurchmesser kleiner als der erste Bohrungsdurchmesser (34) abzüglich des Versatzes (47) und kleiner als der zweite Bohrungsdurchmesser (44) abzüglich des Versatzes (47) ist.

19. System nach Anspruch 17 oder 18, wobei das zweite Verriegelungselement eine Schraube ist.

## Revendications

1. Clou intramédullaire (10), le clou comprenant :
un axe longitudinal du clou (12),
une première partie latérale (15),
une seconde partie latérale (16) opposée à la première partie latérale,
un alésage longitudinal (20),
un premier alésage (30) avec un premier axe d'alésage (32) dans lequel le premier alésage est formé dans la première partie latérale et communique avec l'alésage longitudinal, et
un second alésage (40) avec un second axe d'alésage (42), dans lequel le second alésage est formé dans la seconde partie latérale et communique avec l'alésage longitudinal,
dans lequel le premier axe d'alésage et le second axe d'alésage sont agencés de sorte qu'un seul élément de verrouillage peut être inséré à travers à la fois le premier alésage et le second alésage,
**caractérisé en ce que** le premier axe d'alésage et le second axe d'alésage sont disposés avec un décalage (47) entre eux.

2. Clou intramédullaire (10) selon la revendication 1, dans lequel le premier alésage (30) comprend un premier diamètre de l'alésage (34) et le second alésage (40) comprend un second diamètre de l'alésage (44), dans lequel le décalage (47) est inférieur à 20 pour cent du premier diamètre de l'alésage (34) et est inférieur à 20 pour cent du second diamètre de l'alésage (44).

3. Clou intramédullaire (10) selon la revendication 2, dans lequel le premier diamètre de l'alésage (34) est égal au second diamètre de l'alésage (44).

4. Clou intramédullaire (10) selon l'une quelconque des revendications 1 à 3, dans lequel le premier axe d'alésage (32) est disposé parallèlement au second axe d'alésage (42).

5. Clou intramédullaire (10) selon l'une quelconque des revendications 1 à 4, dans lequel le premier axe d'alésage (32) et le second axe d'alésage (42) sont disposés avec un décalage (47) dans une direction de l'axe du clou (12).

6. Clou intramédullaire (10) selon l'une quelconque des revendications 1 à 5, dans lequel le premier axe d'alésage (32) et le second axe d'alésage (42) sont disposés avec un décalage (47) dans une direction perpendiculaire à l'axe du clou (12).

7. Clou intramédullaire (10) selon l'une quelconque des revendications 1 à 6, dans lequel le premier axe d'alésage (32) et le second axe d'alésage (42) sont disposés perpendiculairement à un plan contenant l'axe du clou (12).

8. Clou intramédullaire (10) selon l'une quelconque des revendications 1 à 7, dans lequel au moins l'un du premier axe d'alésage (32) et du second axe d'alésage (42) coupent l'axe de clou (12).

9. Clou intramédullaire (10) selon l'une quelconque des revendications 1 à 8, dans lequel le premier diamètre de l'alésage (34) est inférieur à 60 pour cent d'un diamètre du clou (14), et dans lequel le second diamètre de l'alésage (44) est inférieur à 60 pour cent du diamètre du clou (14).

10. Clou intramédullaire (10) selon l'une quelconque des revendications 1 à 9, dans lequel un diamètre de l'alésage longitudinal (24) est inférieur à 60 pour cent d'un diamètre du clou (14).

11. Clou intramédullaire (10) selon l'une quelconque des revendications 1 à 10, dans lequel un axe de l'alésage longitudinal (22) est congruent avec l'axe longitudinal du clou (12).

12. Système comprenant :
un clou intramédullaire (10) selon l'une quelconque des revendications 1 à 11, et
un premier élément de verrouillage (50) ayant un diamètre du premier élément de verrouillage (54),
dans lequel le diamètre du premier élément de verrouillage est plus petit que le diamètre du premier alésage (34) et plus grand que le diamètre du premier alésage (34) moins le décalage (47), et est plus petit que le diamètre du second alésage (44) et plus grand que le diamètre du second alésage moins le décalage (47).

13. Système selon la revendication 12, dans lequel le premier élément de verrouillage (50) est une vis.

14. Système selon la revendication 13, dans lequel la vis (50) comporte un premier filetage de vis (57) qui est formé de telle sorte que le premier filetage de vis peut s'engager par frottement à la fois avec le premier alésage et le second alésage.

15. Système selon l'une quelconque des revendications 12 à 14, dans lequel le premier élément de verrouillage (50) comporte un matériau du premier élément de verrouillage et le clou (10) comporte un matériau du clou, dans lequel le matériau du premier élément de verrouillage est plus dur que le matériau du clou.

16. Système selon l'une quelconque des revendications 12 à 14, dans lequel le premier élément de verrouillage (50) comporte un matériau du premier élément de verrouillage et le clou (10) comporte un matériau du clou, dans lequel le matériau du clou est plus dur que le matériau du premier élément de verrouillage.

17. Système selon l'une quelconque des revendications 12 à 16, comprenant en outre un second élément de verrouillage (60) ayant un diamètre du second élément de verrouillage (64), dans lequel le diamètre du second élément de verrouillage est plus petit que le diamètre du premier alésage (34) moins le décalage (47) et plus petit que le diamètre du second alésage (44) moins le décalage (47).

18. Système comprenant:
un clou intramédullaire (10) selon l'une quelconque des revendications 1 à 11, et
un second élément de verrouillage (60) ayant un diamètre du second élément de verrouillage (64), dans lequel le diamètre du second élément de verrouillage est plus petit que le diamètre du premier alésage (34), moins le décalage (47) et plus petit que le diamètre du second alésage (44) moins le décalage (47).

19. Système selon la revendication 17 ou 18, dans lequel le second élément de verrouillage est une vis.
